# EUROPEAN PATENT SPECIFICATION

(11) **EP 1 077 716 B1**
(45) Date of publication and mention of the grant of the patent: **27.07.2005**
(21) Application number: 99924252.2
(22) Date of filing: 14.05.1999
(51) Int. Cl.: A61K 39/00, A61K 39/385, A61K 38/00, A61K 31/44, A61K 31/415, A61K 31/425

(54) **PREPARATION OF A MEDICAMENT FOR THE TREATMENT OF GASTROESOPHAGEAL REFLUX DISEASE**
VORBEREITUNG EINES MEDIKAMENTS ZUR BEHANDLUNG VON REFLUXÖSOPHAGITIS
PREPARATION DES MEDICAMENTS POUR LE TRAITEMENT DE REFLUX GASTRO-OESOPHAGIEN PATHOLOGIQUE

(30) Priority: 15.05.1998 US 85610 P
(43) Date of publication of application: 28.02.2001
(73) Proprietor: APHTON CORPORATION, Miami, FL 33130 (US)
(72) Inventor: GEVAS, Philip, C., Key Biscayne, FL 33149 (US); GRIMES, Stephen, Davis, CA 95616 (US); KARR, Stephen, Davis, CA 95616 (US); MICHAELI, Dov, Larkspur, CA 94939 (US)
(74) Representative: Hale, Stephen Geoffrey
(86) International application number: PCT/US1999/010734
(87) International publication number: WO 1999/059612

(56) References cited:
- US-A- 5 023 077
- US-A- 5 468 494
- US-A- 5 609 870
- HALTER F ET AL: "EVALUATION OF A MONOCLONAL ANTI-GASTRIN ANTIBODY AS A TOOL FOR IMMUNONEUTRALIZATION OF GASTRIN DURING OMEPRAZOLE TREATMENT IN THE RAT" GASTROENTEROLOGY, vol. 96, no. 5 PART 2, 1989, page A194 XP009004160 ISSN: 0016-5085
- BUDAVARI S., The Merck Index (11th Ed.), Rahway, N.J.: Merck & Co., 1989, page 1082, XP002921541.

## Description

The present invention relates to a medicament for the treatment of gastroesophageal reflux disease.

### BACKGROUND OF INVENTION

Gastroesophageal reflux disease ("GERD") is a common and chronic disorder which requires long-term, even lifelong, therapy. GERD is commonly known as heartburn, and is characterized by a retrosternal burning sensation, and regurgitation of the stomach contents. About 40% of adults in the United States have experienced occurrences of the disease, and approximately 10% have daily troubling symptoms.

GERD occurs when there is an abnormally prolonged contact time between the esophageal mucosa and refluxate, which is believed to be primarily gastric acid (DeVault, et al., *Mayo Clinic Proc*. 69:867-876, 1994 and Redmond, et al. In "Gastroesophageal Reflux Disease" Ronald Hinder ed., RG. Landes Co., Ch. 1, pages 1-6, 1993). The regurgitation of the gastric contents and duodenal juice is believed to be due either to an incompetent lower esophageal sphincter or more frequently to an inappropriate sphincter relaxation at the time of transfer of the stomach contents between stomach and small intestine. The resultant reflux of acid and other materials from the stomach may induce pain or damage the esophageal mucosa. This damage to the esophageal mucosa may lead to esophagitis, which is characterized by inflammation of the esophageal mucosa, bleeding, cytological changes, peptic esophageal stricture, esophageal ulcer and Barrett's metaplasia, depending on the severity of the disease.

Gastric acid is produced by parietal cells in the stomach upon stimulation by acetylcholine, histamine and gastrin following the binding of each of these compounds with specific receptors on the surface of the cells. The peptide hormone gastrin is produced by mucosal cells in the stomach. Gastrin is secreted into the blood stream and is the most potent stimulant of acid secretion by the parietal cell. Gastrin is present in two molecular forms, heptadecagastrin (G17) and tetratriacontagastrin (G34). G17 is the primary stimulator of meal-induced gastric acid secretion and is 1500 times more potent than histamine, accounting for 60% of the gastrin-mediated acid release. It has also been found that in GERD patients having an abnormal sphincter, the postprandial levels of gastrin are twice those of a normal person and remain high, beyond 3 hours after the meal (Wetscher, et al. In *Gastroesophageal Reflux Disease,* R.A. Heinder, ed. R.G. Landes Co., Ch. 2, pages 7-29, 1993).

Normal esophageal pH is greater than pH 4. The acid refluxate from the stomach lowers the pH value of the esophagus to less than 4, which results in damage to the esophageal mucosa and the development of GERD. In normal individuals, acidic refluxate is cleared by elimination of the refluxate by peristalsis of the esophagus and by neutralization of the acid with the bicarbonate produced by submucosal esophageal glands, and the bicarbonate present in swallowed saliva. In GERD patients, these mechanisms of acid neutralization are not sufficient to restore the normal esophageal pH values and prevent mucosal damage, since reflux of stomach contents occurs more frequently, and for a more prolonged period of time, than in normal individuals (Booth, et al., *Arch. Surg.* 96: 731-734, 1968 and Demeester, at al., *Ann. Surg.* 184: 459-470, 1976). Since it is not medically practical to alter the esophageal acid neutralization mechanisms, GERD therapies are directed to raising the pH of the stomach contents.

Currently, various therapies are available for the treatment of GERD. Historically, the medical treatment for GERD consisted of using antacids as acid neutralizing agents or anti-refluxants, such as alginates, for alleviating an acute onset of the disease. However, these treatments are not effective for the therapy of chronic and severe symptoms of GERD. Systemic medications currently used for treating GERD include the histamine receptor antagonists, cimetidine and ranitidine, which are acid-suppressive agents directed to the inhibition of the four histamine type 2 ("H₂") receptors. These agents prevent the normal binding of histamine, thereby inhibiting the parietal cell from secreting gastric acid and thus, they increase the pH of the stomach contents. The most commonly used histamine H₂ - antagonists are cimetidine hydrochloride (Tagamet®, SmithKline Beecham Pharmaceuticals), ranitidine hydrochloride (Zantac®, Glaxo Pharmaceuticals), fomatidine (Pepcid®, Merck & Co.) and nizatidine (Eli Lilly & Co.). The use of these H₂ antagonists is the standard treatment of acid-caused peptic disorders including GERD, since surgery, a more radical approach, is usually contraindicated.

Despite the widespread acceptance of histamine H₂ receptor blockers, controlled studies on GERD patients treated with these acid inhibiting compounds have yielded variable results on the healing of esophagitis and persistent symptomatic responses, such as continued acid production in the stomach. Studies using cimetidine and ranitidine in GERD patients, at doses and durations that had been proven effective in healing peptic ulcers, were not effective in GERD (Sabesin et al. *Arch. Intern. Med.* 151:2394, 1991). At higher doses and duration of the H₂-antagonist therapy (400-800mg and 150-300mg twice daily, respectively, for cimetidine and ranitidine), approximately 50-70% (mean, 61%) of patients had symptomatic relief of GERD, and 0 to 82% (mean, 48%) had healing of their esophagitis as endoscopically determined (DeVault, et at. *ibid*; Koelz, H.R. Scand. J. Gastroenterol. 24:25-26, 1989 and Fennerty and Sampliner, *Arch. Intern. Med*. 151:2365-2366, 1991).

The healing of ulcerated or eroded esophageal mucosa requires a longer and more profound acid suppression than is necessary in treating other gastrointestinal ulcers. In patients in whom the symptoms of GERD disappeared after an effective treatment with histamine blockers, the symptoms of the disease reappeared soon after the treatment was discontinued (Antonson, et al. *Gastroenterology* 98: A16, 1990 and Bardhan, et al. *Gastroenterology* 98: A18, 1990).

Many patients with severe GERD hypersecrete gastric acid and may require high doses of H₂ antagorusts, which become problematic in terms of patient compliance and long term use of these agents. The high doses of H₂ blockers when given to patients for a long period of time may cause undesirable side effects such as, blood pressure and heart problems. The increase in the effective dosage required to bring about relief of GERD symptoms results in very costly therapy. Although treatments of esophagitis vary widely depending on the severity of the disease, the more severe, high-grade types of the disease respond poorly to standard doses of histamine blockers. Approximately 50% or more of patients with GERD do not respond to histamine H₂ antagonist therapy and still require some other form of treatment. In addition, the effective treatment of GERD not only depends on increasing the concentration of histamine blockers or the hydrogen pump inhibitors which have also been found to be effective in the treatment of GERD, but effective dosaging must be also frequent, since the compounds have limited transient time in the patient and must be given in some situations approximately 4 times daily. In a significant number of cases, the patient is not responsive to H₂ blockers.

Proton pump inhibitors omeprazole (Astra AB), or anti-H⁺/K⁺-ATPase enzyme inhibitory compound, as well as its analogue, lansoprazole, (Takeda Chemicals) or pantoprazole (Byk Gulden), which inhibit acid secretion in the stomach by inhibiting the proton (hydronium ion) pump mechanism for producing hydrochloric acid in the parietal cell, have been found to be more effective than histamine H₂ blockers in alleviating the symptoms of GERD esophagitis. The resulting increase in pH induced by omeprazole leads to approximately 62-94% (mean, 83%) in symptomatic relief, and a healing of the esophagitis occurs in 71-96% (mean, 78%) of the patients in 4-8 weeks of treatment for GERD, almost twice that of ranitidine (DeVault et al. *ibid*; Zeitoun, P. *Scand. J. Gastroenterology* 166 (Suppl.): 83, 1989). A disadvantage of using omeprazole, lansoprazole or pantoprazole, similar to the case with histamine blockers, is that the compound must be administered at higher doses (20 mg, twice daily or 40 mg once daily) than the dosages required to treat gastric and duodenal ulcers (20mg, once daily), and for a longer period of time, in order to effectively treat GERD.

Furthermore, the prolonged use of histamine blockers or omeprazole for the treatment of GERD results in an increase in serum gastrin levels (2 to 4 times the basal rate). It has been suggested that the increase in gastrin levels could lead to undesirable side effects, such as dangerous trophic effects on the human gastric mucosa (Festen, et al. *Gastroenterology* 87: 1030-1034, 1984; Jansen, et al. *Gastroenterology* 99: 621-628, 1990 and Sontag, et al. *Gastroenterology* 102-109, 1992).

Co-assigned U.S. Patents No. 5,023,077 and No. 5,609, 870 disclose immunogenic compositions useful for controlling gastrin levels in a patient by generating anti-gastrin antibodies. Thus, U.S. Patent No. 5,023,077 and No. 5,609,870 disclose that the immunogenic compositions are useful for the treatment of gastric and duodenal ulcers and gastrin-induced or-responsive cancers.

Halter et al, **1989** *Gastroenterology*, 96(5)2:A194 teaches the use of monoclonal anti-gastrin antibodies for lowering elevated serum gastrin levels following omeprazole treatment. Specifically, Halter discusses artificially inducing hypergastrinemia in rats in intravenous infusion of 500 ng/kg of human gastrin-17. Halter notes that high gastrin binding capacities were maintained in the plasma throughout the study in the Mab-treated animals. Halter concludes that the Mab is capable of binding considerable amounts of plasma gastric acid and thus may be a useful tool for prolonged immunoneutralization of endogenous gastrin.

There remains a need in the art for additional methods and compositions for the successful therapy of GERD.

### SUMMARY OF THE INVENTION

The present invention relies on the combination of reducing gastric acid in the stomach by inhibiting the enzyme responsible for gastric acid production or secretion of gastric acid and immunologically reducing or preventing the increase of circulating gastrin. It is an object of the present invention to use anti-gastrin immunogenic compositions in the therapy of GERD in combination with administering effective doses of a proton pump inhibitor or H₂ antagonist so as to substantially raise the gastric pH while preventing elevated levels of circulating blood gastrin hormone.

Accordingly, the present invention provides the use in the preparation of a medicament for the treatment of gastroesophageal reflux disease in a mammal of
(a) an effective amount of an immunogenic composition comprising a peptide comprising the amino terminal domain of gastrin-17 conjugated to an immunogenic carrier, said peptide inducing anti-gastrin antibody levels in the serum of said mammal, and
(b) an effective amount of an agent selected from a histamine H₂ antagonist and a proton pump inhibitor, wherein treatment with said agent can be reduced or discontinued when said serum anti-gastrin antibody levels are within 10 to 300 pmole/ml.

This invention is thus directed to the treatment of GERD by gastric acid suppression by administration of a proton pump inhibitor or H₂ blocker together with the immunological reduction of circulating gastric hormone by neutralization of heptadecagastrin G17, or both G17 and tetratriacontagastrin G34, *in situ* by an immunogenic composition against gastrin.

Preferably, omeprazole is used as proton pump inhititor and the immunogenic composition induces anti-gastrin antibodies that bind to gastrin G17.

It is preferred to keep the frequency of anti-gastrin immunogen parenteral administration to a patient suffering from GERD at a single effective dose or at least at only a few doses thereof.

It may be preferred to pre-treat the GERD patient with gastrin immunogen (a) before administering the gastric acid producing enzyme inhibitor (i.e. proton pump inhibiting compound) (b).

In one embodiment, the invention concerns a combination therapy with a histamine H₂ antagonist, such as ranitidine, cimetidine, fomatidine or nizatidine, or a proton pump inhibitor, such as omeprazole or lansoprazole, using standard dosing procedures for H₂ antagonist or proton pump inhibitor, respectively, as described by the art. In the preferred combination therapy using the invention, a patient is actively immunized with an immunological composition comprising gastrin 17(1-9)- h(G17)ser9-Diphtheria Toxin (see U.S. Patent Nos. 5,023,077 and 5,468,494 (co-assigned). Once the patient is immunized, histamine H₂ antagonist or proton pump inhibitor therapy is administered for 2-12 weeks or until the desired serum anti-gastrin 17 antibody titer is reached. The combination therapy provides a more effective method for controlling acid output by the stomach, since acid production is thus controlled by two independent mechanisms, which results in a more effective method for treating GERD, including the more severe cases of the disease. In addition, the therapy would be a less costly method for treating GERD, without the problems with patient compliance associated with long term standard therapies. Furthermore, the high gastrin levels associated with standard therapies, particularly with omeprazole, are neutralized, and thus, the undesirable side effects are reduced.

Use of this invention for treating GERD permits a reduced dosage of the acid reducing agent both at the acid producing level as well as the acid production stimulating level (gastrin). This reduction of dosages is desirable in the usually prolonged treatment.

### BRIEF DESCRIPTION OF THE FIGURES

**FIGURE 1** illustrates experimental data concerning the percentage of time that the gastric contents remain above pH 3 in different groups of pigs treated with ranitidine (4 animals), omeprazole (5 animals) and hG17(1-9) (4 animals), as compared to six (6) control animals.
**FIGURE 2** illustrates the percentage of time that the gastric contents remain above pH 4 in a group of untreated (control) pigs (5) and groups of pigs treated with human gastrin 17(1-9)Ser9-Diphtheria Toxin (4 animals), ranitidine (4 animals) and omeprazole (5 animals) as described in **Figure 1.**
**FIGURE 3** depicts the baseline median pH of the gastric contents of a group of six (6) untreated (control) pigs and groups of four (4) pigs treated with human G17(1-9)Ser9-Diphtheria Toxoid, three (3) pigs with ranitidine and five (5) pigs with omeprazole as described in **Figure 1.**

### DETAILED DESCRIPTION OF THE INDENTION

The present invention has utility in relation to a novel combination of methods for the treatment of gastroesophageal reflux disease. The combined method on the one hand comprises inhibiting the normal binding of the hormone gastrin 17 to its physiological receptor by actively immunizing the patient against his or her own gastrin 17 hormone. Additionally, the hormone gastrin 34 can be neutralized by active or passive immunization with G34 or C-terminal G17 peptide fragment. On the other hand, the method provides inhibition of production of gastric acid either by proton pump inhibition or H₂ receptor blockage.

The invention has utility in relation to a novel immunological.method for the treatment of gastroesophageal reflux disease using a peptide immunogen which raises sufficient gastrin 17 antibody levels in a patient so as to affect the binding of the gastrin 17 to its physiological receptors in the patient and raise the pH of the stomach. Gastric acid secretion in the stomach can thus be controlled. The pH of the stomach contents is simultaneously raised to a sufficient pH level, e.g., greater than pH 3 for a prolonged and sufficient period of time to alleviate the GERD symptoms and heal the acid-induced esophagitis. By use of the invention, anti-G17 antibodies may be induced in the patient by active immunization with peptide immunogens which comprise a G17 immunogen conjugated to an immunogenic carrier. The antibodies raised in the patient by the immunogens selectively and specifically bind gastrin hormone G17 or both G17 and G34 and neutralize and inhibit separately or together the normal binding of gastrin G17 or both G17 and G34 to its receptors in the parietal cells, thereby controlling acid output in the stomach and preventing gastric acid damage of the esophageal mucosa during regurgitation.

A preferred embodiment using the invention provides a single administration of an active gastrin 17 immunogen, which has several advantages over the standard therapies of the art for treating GERD in that problems with patient compliance and undesirable side effects as a result of the therapy are eliminated. Other advantages of using the immunological methods for the treatment of GERD include the use of a limited number of administrations. A single primary administration with appropriately spaced boosters may last for approximately 6 months to a year. Another advantage is that, in a combination therapy with H₂ agonists or proton pump inhibitors, effective anti-gastrin 17 antibody titers can be maintained by occasional booster shots while the gastric acid inhibitor dosing is reduced or discontinued. Another advantage of the use of this invention is that the maintenance of antigastrin antibody titers reduces or prevents excessive levels of gastrin in hypogastrinemia which would otherwise result from administration of a proton pump inhibitor or H₂ blocker. A booster shot of the immunological composition prolongs anti-gastrin 17 immunity and gastric acid suppression. Still another advantage of this method is that the immunization allows a sufficient time for the esophagitis to completely heal. Additionally, no surgery is required. Yet another advantage is that combination therapy is more useful for treating severe cases of GERD, without causing undesirable side effects, since excess serum gastrin 17 peptides are physiologically neutralized. In patients where the GERD condition is alleviated, discontinuation of the booster dose may result in resumption of normal gastrin levels.

According to the invention, an immunogen is prepared using peptides that mimic the amino terminal end of gastrin 17. Immunogens and immunogenic compositions suitable for use in the invention are described in U.S. Patent No. 5,023,077, U.S. Patent No. 5,469,494 and U.S. Patent No. 5,609,870. U.S. Patent Nos. 5,023,077, 5,469,494, and 5,609,870 disclose compositions containing anti-gastrin 17 immunogens as well as anti-gastrin 34 immunogens and methods of using these compositions for the treatment of gastric and duodenal ulcers and gastrin responsive cancers.

In use of the present invention, effective dosages ranging from 0.1 mg to 5g of the immunogenic composition may be administered for the treatment of GERD combined with 10-80mg daily dose of omeprazole. An effective dosage of the immunogenic composition is capable of eliciting an immune response in a patient and inducing antibody titer against human gastrin 17 within 1-3 months after immunization.

Effective treatment of GERD according to this method results in maintenance of the pH of the stomach contents above pH 3 or 4, and for a more prolonged period of time than with H₂ antagonist therapy. Maintenance of the stomach pH above 3 or 4 is essential in the treatment of GERD, since refluxate material having a pH below 2.0 causes esophagitis by protein denaturation and cell damage, and pH values below 2.5 triggers painful episodes in a patient. When the pH is maintained above 2.5, pain perception is almost nonexistent (Smith, et al *Gastroenterology* 96: 683-689, 1989) and damage to the esophageal wall is minimized.

The immunogens and immunogenic compositions provided by use of the invention typically induce specific antibody responses after a single administration. However, it may take several weeks or months for antibody titers to rise to the desired levels effective for the treatment of GERD.

Combination therapy with a histamine H₂ antagonist, such as ranitidine, cimetidine, fomatidine and nazatidine, or a proton pump inhibitor, such as omeprazole or lansoprazole, is designed so that a GERD patient is immunized with an immunogenic composition of the invention, and administration of H₂ antagonist is provided on a daily basis, at least once a day for the first 2-12 weeks of treatment or until the desired serum level of anti-gastrin 17 antibodies is obtained.

Desired anti-gastrin 17 serum levels range from 10 to 300 pmole/ml. Once the desired serum levels of anti-gastrin 17 antibody titer are obtained as measured by ELISA or RIA, the non-immunological gastric acid inhibiting drug portion of the combination therapy may be reduced or discontinued.

In the following Examples, the anti G17 immunogenic composition, 150mg ranitidine and 60mg omeprazole were administered to pigs and the resulting changes in the pH of the stomach contents before and after treatment were measured. Specifically, following the stomach pH measurements of the untreated control state of each pig, the stomach pH of the same pigs was measured after the animals were treated with either ranitidine, or composition of human gastrin 17(1-9)-h(G17)ser9-Diphtheria Toxoid (Gastrimmune), or omeprazole administered individually and at different times in each of four animals (pigs).

### Example 1

Gastrin neutralization was achieved by using the immunological composition Gastrimmune which is composed of the amino terminal domain of gastrin-17 linked, via an amino acid or peptide spacer to diphtheria toxoid which acts as the immunogenic carries. The antibodies raised by virtue of the design of the immunogen, cross-reacted with both amidated and glycine-extended gastrin-17, two known proliferative forms of gastrin.

Serum antibody titers rose within 2 weeks of the initial immunization to levels with an antigen binding capacity of > 10⁻⁹M. The presence of anti-gastrin antibodies within the serum of Gastrimmune-immunized mice was confirmed by using an ELISA. As expected, no bound gastrin levels were detected in animals immunized with control immunogen.

### Example 2

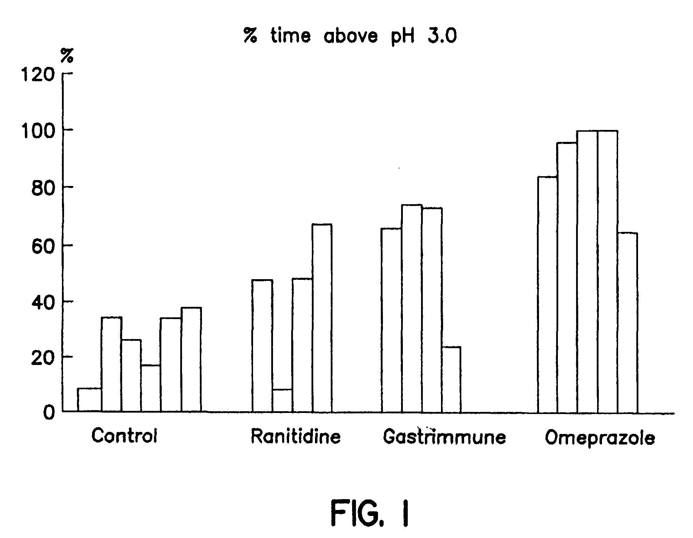

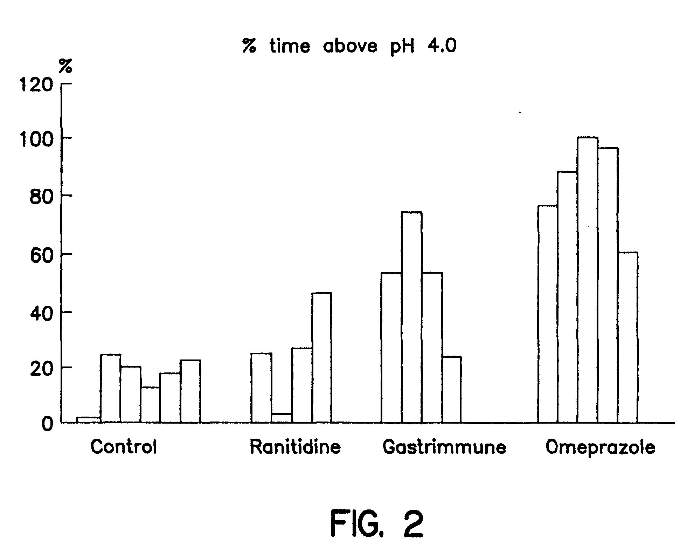

As can be seen in **FIG. 1** and **FIG. 2,** the pH of the stomach contents remained above pH 3 or 4 in anti-gastrin 17 immunized pigs for a longer period of time than in the pigs treated with ranitidine. In omeprazole treated pigs the stomach pH was maintained above pH 3 or 4 for a longer period of time than pigs which were treated with ranitidine and anti-G17 immunized pigs.

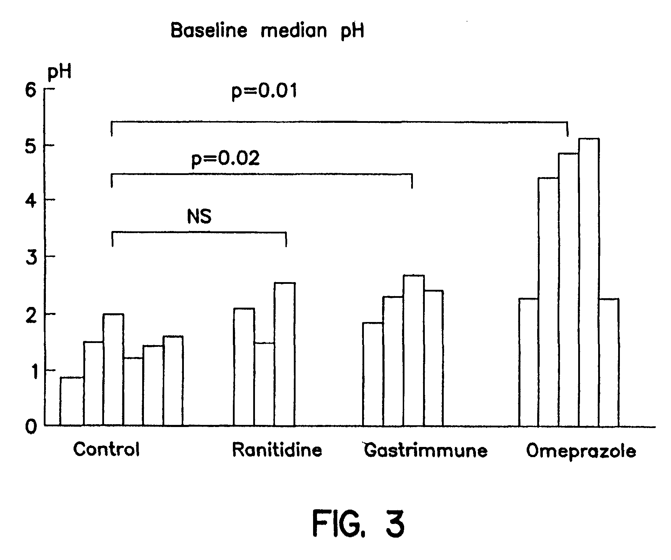

In addition, **FIG. 3** shows the median pH exhibited by the stomach contents of control pigs when compared to ranitidine, anti-G17 immunization and omeprazole treatment. The data shows that the stomach pH is maintained at higher levels in pigs than those treated with ranitidine or anti-G17 immunization therapy. Anti-G17 immunized pigs had a median pH higher than ranitidine treated pigs.

Treatment of the pigs with ranitidine was less effective in preventing acid output from the stomach. Omeprazole treatment highly inhibited acid output. A single administration of anti-gastrin 17 immunization inhibited stomach acid output at a level of effectiveness between ranitidine and omeprazole, and sufficient to reduce the stomach acid output levels and increase the stomach pH for the effective treatment of GERD.

A treatment which combines the gastric acid secretion with proton pump inhibitors or H₂ histamine blockers with the novel immunization by e.g. Gastrimmune can thus result in maintaining favorably raised pH in the stomach. Furthermore, the treatment with occasional, effective boosters of the antigastrin immunogenic composition can eventually, possibly within a few months, obviate any additional treatment with the anti-acid secretion drugs, such as e.g. omeprazole or ranitidine.

One of the possible advantages of the administration of a proton pump inhibitor or H₂ blocker after immunization with an antigastrin immunogen, as described, resides in the use of lower amounts of the proton pump inhibitor or H₂ blocker for effective lowering of gastrin acid secretion or raising of stomach pH to about 3-4.

### Example 3

The human patient suffering from GERD is immunized with 200 µg - 400 µg of primary i.v. inoculation of G17 (1-9) Ser: DT immunogen composition. After 2 weeks a booster of 100 - 200 µg of the G17 (1-9) Ser : DT composition is similarly administered. When the anti-G17 titer has reached a level of about 10-300 pmole/ml sufficient to lower the serum gastrin level to near normal with a concomitant lowering of gastric acid secretion, about 10-20 mg oral omeprazole preparation is administered daily to further reduce or stabilize the gastric secretion at a level which essentially eliminates or substantially ameliorates the GERD symptoms.

### Example 4

Immunogens capable of inducing specific immune responses to either G17 or to G34 were prepared by standard solid state synthesis methods. Each peptide was characterized as to amino acid content and purity.

Peptides with the following amino acid sequences were synthesized:
Peptide 1 - Human G17(1-6) ("hG17(6)"): pGlu-Gly-Pro-Trp-Leu-Glu-Arg-Pro-Pro-Pro-Pro-Cys
Peptide 2 - Human G17(1-5) ("hG17(5)"): pGlu-Gly-Pro-Trp-Leu-Arg-Pro-Pro-Pro-Pro-Cys
Peptide 3 - Human G17(1-4) ("hG17(4)"): pGlu-Gly-Pro-Trp-Arg-Pro-Pro-Pro-Pro-Cys
Peptide 4 - Rat G17(1-6) ("rG17(6)"): pGlu-Arg-Pro-Pro-Leu-Glu-Arg-Pro-Pro-Pro-Pro-Cys
Peptide 5 - Human G34(1-6) ("hG34(6)"): pGlu-Leu-Gly-Pro-Gln-Gly-Arg-Pro-Pro-Pro-Pro-Cys
Peptide 6 - Human G34(13-22) ("hG34/G17 combination"): Asp-Pro-Ser-Lys-Lys-Gln-Gly-Pro-Trp-Leu-Pro-Pro-Pro-Pro-Cys

Each of these peptides were conjugated to amino groups present on a carrier such as Diphtheria toxoid ("DT") via the terminal peptide cysteine residue utilizing heterobifunctional linking agents containing a succinimidyl ester at one end and maleimide at the other end of the linking agent.

To accomplish the linkage between any of Peptides 1-6 above and the carrier, the dry peptide was dissolved in 0.1 M Sodium Phosphate Buffer, pH 8.0, with a thirty molar excess of dithiothreitol ("DTT"). The solution was stirred under a water saturated nitrogen gas atmosphere for four hours. The peptide containing reduced cysteine was separated from the other components by chromatography over a G10 Sephadex column equilibrated with 0.2 M Acetic acid. The peptide was lyophilized and stored under vacuum until used. The carrier was activated by treatment with the heterobifunctional linking agent, e.g., Epsilon-maleimidocaproic acid N-hydroxysuccinimide ester, ("EMCS"), in proportions sufficient to achieve activation of approximately 25 free amino groups per 10⁵ molecular weight of carrier. In the specific instance of diphtheria toxoid, this amounted to the addition of 6.18 mg of EMCS (purity 75%) to each 20 mg of diphtheria toxoid.

Activation of diphtheria toxoid was accomplished by dissolving each 20 mg aliquot of diphtheria toxoid in 1 ml of 0.2 M Sodium Phosphate Buffer, pH 6.45. Aliquots of 6.18 mg EMCS were dissolved into 0.2 ml of Dimethyl Formamide ("DMF"). Under darkened conditions, the EMCS was added dropwise in 50 microliter ("ul") amounts to the DT with stirring. After 2 hours of incubation in darkness, the mixture was chromatographed on a G50 Sephadex column equilibrated with 0.1 M Sodium Citrate buffer, pH 6.0, containing 0.1 mM EDTA.

Fractions containing the EMCS activated diphtheria toxoid were concentrated over a PM 10 ultrafiltration membrane under conditions of darkness. The protein content of the concentrate was determined by either the Lowry or Bradford methods. The EMCS content of the carrier was determined by incubation of the activated carrier with cysteine-HCI followed by reaction with 10 mM of Elman's Reagent 5,5'dithio-bis (2-nitrobenzoic acid) 10 mM. The optical density difference between a blank tube containing cysteine-HCl and the sample tube containing cysteine-HCl and carrier was translated into EMCS group content by using the molar extinction coefficient of 13.6 x 10³ for 5-thio-2-nitro benzoic acid at 412 nm.

The reduced cysteine content (-SH) of the peptide was also determined utilizing Elman's Reagent. Approximately 1 mg of peptide was dissolved in 1 ml of nitrogen gas saturated water and a 0.1 ml aliquot of this solution was reacted with Elman's Reagent. Utilizing the molar extinction coefficient of 5-thio-2-nitro-benzoic acid (13.6 x 10³), the free cysteine -SH was calculated. An amount of peptide containing sufficient free -SH to react with each of the 25 EMCs activated amino groups on the carrier was dissolved in 0.1 M Sodium Citrate Buffer, pH 6.0, containing 0.1 mM EDTA, and added dropwise to the EMCS activated carrier under darkened conditions. After all the peptide solution had been added to the carrier, the mixture was incubated overnight in the dark under a water saturated nitrogen gas atmosphere.

The conjugate of the peptide linked to the carrier via EMCS is separated from other components of the mixture by chromatography over a G50 Sephadex column equilibrated with 0.2 M Ammonium Bicarbonate. The conjugate eluted in the column void volume is lyophilized and stored desiccated at 20°C until used.

The conjugate may be characterized as to peptide content by a number of methods known to those skilled in the art including weight gain, amino acid analysis, etc. Conjugates of these peptides and diphtheria toxoid produced by these methods were determined to have 20-25 moles of peptide per 10⁵ MW of carrier and all were considered suitable as immunogens for immunization of test animals.

### Example 5

Peptide hG17(1-9)-Ser9 was prepared by standard solid state synthesis methods. That peptide contains an amino terminal immunomimic of hG17 followed by a carboxy terminal spacer. This peptide comprises a 9 amino acid immunomimic of hG17 (pGlu-Gly-Pro-Trp-Leu-Glu-Glu-Glu-Glu-) followed by the "Ser" spacer (-Ser-Ser-Pro-Pro-Pro-Pro-Cys) attached to amino acid number 9 of the hG17 immunomimic.

The peptide was conjugated to amino groups present on the Diphtheria Toxoid ("DT") immunogenic carder via the terminal peptide cysteine residue utilizing heterobifunctional linking agents containing a succinimidyl ester at one end and maleimide at the other end of the linking agent essentially as described in Example 4.

The immunogenic constructs of this invention include an aminoterminal (1-9) G17 peptide or an aminoterminal (1-6) G34 peptide conjugated via a peptide spacer to an immunogenic carrier. The preferred G17 sequence is pyro-Glu-Gly-Pro-Trp-Leu-Glu-Glu-Glu-Glu and the preferred G34 sequence is pGlu-Leu-Gly-Pro-Gln-Gly-Arg-Pro-Pro-Pro-Pro-Cys. The preferred spacer in both constructs is a Ser-peptide (Ser-Ser-Pro-Pro-Pro-Pro-Cys). The preferred immunogenic carrier is diphtheria toxoid, tetanus toxoid, keylimpet hemocyanin, and bovine serum albumin (BSA). The gastrin immunogen is defined as a conjugate of the pGlu- Gly-Pro-Trp-Leu-Glu-Glu-Glu-Glu peptide sequence, with an amino acid spacer linked to an immunogenic carrier. The preferred gastrin immunogen is defined as a conjugate of the (1-9) amino terminal (pGlu-Gly-Pro-Trp-Leu-Glu-Glu-Glu-Glu) peptide which is linked by peptide spacer to diphtheria toxoid.

## Claims

1. The use in the preparation of a medicament for the treatment of gastroesophageal reflux disease in a mammal of
(a) an effective amount of an immunogenic composition comprising a peptide comprising the amino terminal domain of gastrin-17 conjugated to an immunogenic carrier, said peptide inducing anti-gastrin antibody levels in the serum of said mammal, and
(b) an effective amount of an agent selected from a histamine H2 antagonist and a proton pump inhibitor,
wherein treatment with said agent can be reduced or discontinued when said serum anti-gastrin antibody levels are within 10 to 300 pmole/ml.

2. The use according to claim 1, wherein said immunogenic composition includes a pharmaceutically acceptable carrier.

3. The use according to claim 1 or 2, wherein said peptide is linked through an amino acid spacer to an immunogenic carrier.

4. The use according to claim 2 or 3, wherein said immunogenic carrier is diphtheria toxoid.

5. The use according to claim 2, wherein said composition induces anti-gastrin antibodies that bind to gastrin.

6. The use according to claim 5, wherein said antibodies bind to and neutralise heptadecagastrin G17.

7. The use according to claim 5, wherein said antibodies comprise a mixture of antibodies that bind to and neutralise heptadecagastrin G17 and antibodies that bind to and neutralise tetratriacontagastrin G34.

8. The use according to any of claims I to 7, wherein said agent is a histamine H2 antagonist.

9. The use according to claim 8, wherein said antagonist is selected from ranitidine hydrochloride, cimetidine hydrochloride, fomatidine, and nizatidine.

10. The use according to any of claims I to 7, wherein said agent is a proton pump inhibitor.

11. The use according to claim 10, wherein said inhibitor is selected from omeprazole, lansoprazole and pantoprazole.

12. The use according to any of claims 1 to 11, wherein said immunogenic composition is to be administered before said agent.

13. The use according to any of claims 1 to 12, wherein said agent is to be administered to said mammal until the serum anti-G17 antibody titer is 10-300 pmole/ml.

14. The use according to any of claims 1 to 13, wherein said immunogenic composition is to be administered periodically to maintain said serum anti-gastrin antibody levels.

## Patentansprüche

1. Verwendung
(a) einer wirksamen Menge einer immunogenen Zusammensetzung, umfassend ein die aminoterminale Domäne von Gastrin-17 enthaltendes Peptid konjugiert an einen immunogenen Träger, wobei das Peptid Antigastrin-Antikörperspiegel im Serum eines Säugetiers induziert, und
(b) eine wirksame Menge eines aus einem Histamin-H2-Antagonisten und einem Protonenpumpenhemmer ausgewählten Mittels,
wobei die Behandlung mit dem Mittel abgesenkt oder abgebrochen werden kann, wenn die Antigastrin-Antikörperspiegel im Serum im Bereich von 10 bis 300 pmol/ml liegen,
bei der Herstellung eines Medikaments zur Behandlung von Refluxkrankheit der Speiseröhre in diesem Säugetier.

2. Verwendung nach Anspruch 1, wobei die immunogene Zusammensetzung einen pharmazeutisch unbedenklichen Träger enthält.

3. Verwendung nach Anspruch 1 oder 2, wobei das Peptid über einen Aminosäurespacer an einen immunogenen Träger gebunden ist.

4. Verwendung nach Anspruch 2 oder 3, wobei es sich bei dem immunogenen Träger um Diphtherietoxoid handelt.

5. Verwendung nach Anspruch 2, wobei die Zusammensetzung Antigastrin-Antikörper induziert, die sich an Gastrin binden.

6. Verwendung nach Anspruch 5, wobei sich die Antikörper an Heptadecagastrin G17 binden und dieses neutralisieren.

7. Verwendung nach Anspruch 5, wobei die Antikörper aus einer Mischung von Antikörpern, die sich an Heptadecagastrin G17 binden und dieses neutralisieren, und Antikörpern, die sich an Tetratriacontagastrin G34 binden und dieses neutralisieren, bestehen.

8. Verwendung nach einem der Ansprüche 1 bis 7, wobei es sich bei dem Mittel um einen Histamin-H2-Antagonisten handelt.

9. Verwendung nach Anspruch 8, wobei der Antagonist aus Ranitidinhydrochlorid, Cimetidinhydrochlorid, Fomatidin und Nizatidin ausgewählt ist.

10. Verbindung nach einem der Ansprüche 1 bis 7, wobei es sich bei dem Mittel um einen Protonenpumpenhemmer handelt.

11. Verwendung nach Anspruch 10, wobei der Hemmer aus Omeprazol, Lansoprazol und Pantoprazol ausgewählt ist.

12. Verwendung nach einem der Ansprüche 1 bis 11, wobei die immunogene Zusammensetzung vor dem Mittel verabreicht werden soll.

13. Verwendung nach einem der Ansprüche 1 bis 12, wobei das Mittel dem Säugetier verabreicht werden soll, bis der Anti-G17-Antikörpertiter im Serum 10-300 pmol/ml beträgt.

14. Verwendung nach einem der Ansprüche 1 bis 13, wobei die immunogene Zusammensetzung periodisch verabreicht werden sollte, um die Antigastrin-Antikörperspiegel im Serum aufrechtzuerhalten.

## Revendications

1. Utilisation dans la préparation d'un médicament destiné au traitement de la maladie du reflux gastro-oesophagien, chez un mammifère,
(a) d'une quantité efficace d'une composition immunogène comprenant un peptide comprenant le domaine amino-terminal de la gastrine-17 conjugué à un support immunogène, ledit peptide induisant des taux en anti-corps anti-gastrine dans le sérum dudit mammifère, et
(b) d'une quantité efficace d'un agent choisi parmi un antagoniste de l'histamine H2 et un inhibiteur de la pompe à protons,
dans laquelle le traitement par ledit agent peut être réduit ou arrêté lorsque lesdits taux en anticorps anti-gastrine sériques sont dans la plage de 10 à 300 pmoles/ml.

2. Utilisation selon la revendication 1, dans laquelle ladite composition immunogène comporte un support pharmaceutiquement acceptable.

3. Utilisation selon la revendication 1 ou 2, dans laquelle ledit peptide est fixé par l'intermédiaire d'un espaceur d'acide aminé à un support immunogène.

4. Utilisation selon la revendication 2 ou 3, dans laquelle ledit support immunogène est le toxoïde de la diphtérie.

5. Utilisation selon la revendication 2, dans laquelle ladite composition induit des anticorps anti-gastrine qui se fixent à la gastrine.

6. Utilisation selon la revendication 5, dans laquelle lesdits anticorps se fixent à, et neutralisent, l'heptadécagastrine G17.

7. Utilisation selon la revendication 5, dans laquelle lesdits anticorps comprennent un mélange d'anticorps se fixant à, et neutralisant, l'heptadécagastrine G17 et des anticorps se fixant à, et neutralisant, la tétratriacontagastrine G34.

8. Utilisation selon l'une quelconque des revendications 1 à 7, dans laquelle ledit agent est un antagoniste de l'histamine H2.

9. Utilisation selon la revendication 8, dans laquelle ledit antagoniste est choisi parmi le chlorhydrate de ranitidine, le chlorhydrate de cimétidine, la fomatidine et la nizatidine.

10. Utilisation selon l'une quelconque des revendications 1 à 7, dans laquelle ledit agent est un inhibiteur de la pompe à protons.

11. Utilisation selon la revendication 10, dans laquelle ledit inhibiteur est choisi parmi l'oméprazole, le lansoprazole et le pantoprazole.

12. Utilisation selon l'une quelconque des revendications 1 à 11, dans laquelle ladite composition immunogène est destinée à une administration avant ledit agent.

13. Utilisation selon l'une quelconque des revendications 1 à 12, dans laquelle ledit agent est destiné à une administration audit mammifère jusqu'à ce que le titre d'anticorps anti-G17 sérique soit de 10-300 pmoles/ml.

14. Utilisation selon l'une quelconque des revendications 1 à 13, dans laquelle ladite composition immunogène est destinée à une administration périodiquement pour maintenir lesdits taux en anticorps anti-gastrine sériques.
